# NEW EUROPEAN PATENT SPECIFICATION

(11) **EP 0 572 401 B2**
(45) Date of publication and mention of the opposition decision: **07.11.2007**
(45) Mention of the grant of the patent: 12.01.2000
(21) Application number: 91915104.3
(22) Date of filing: 09.08.1991
(51) Int. Cl.: C12N 15/87, C12N 15/86, A61K 48/00, C12N 5/10, C12N 15/47, C12N 15/48

(54) **VIRAL PARTICLES HAVING ALTERED HOST RANGE**
VIRUSPARTIKEL MIT VERAENDERTEM WIRTSPEKTRUM
PARTICULES VIRALES AGISSANT SUR UNE GAMME D'HOTES MODIFIEE

(30) Priority: 19.02.1991 US 658632
(43) Date of publication of application: 08.12.1993
(73) Proprietor: The Regents of the University of California, Oakland, CA 94607-5200 (US); Oxford Biomedica (UK) Ltd, Robert Robinson Avenue Oxford Science Park Oxford, OX4 4GA (GB)
(72) Inventor: YEE, Jiing-Kuan, Del Mar, CA 92014 (US); EMI, Nobuhiko, 3-102, Show-ku Nagoya 466 (JP); FRIEDMAN, Theodore, La Jolla, CA 92037 (US); JOLLY, Douglas, J., La Jolla, CA 92037 (US)
(74) Representative: Campbell, Patrick John Henry
(86) International application number: PCT/US1991/005699
(87) International publication number: WO 1992/014829

(56) References cited:
- EP-A- 0 243 204
- WO-A-89/01516
- WO-A-90/05538
- WO-A-90/12087
- WO-A-90/15141
- WO-A-91/02508
- WO-A-91/06658
- EMI N. ET AL.: 'Pseudotype Formation of Murine Leukemia Virus with the G Protein of Vesicular Stomatitis Virus' JOURNAL OF VIROLOGY vol. 65, no. 3, March 1991, pages 1202 - 1207, XP000283118
- ZAVADA H. ET AL.: 'Vesicular Stomatitis Virus Phenotypically Mixed with Retroviruses: an Efficient Detection Method' ACTA VIROLOGICA vol. 27, no. 2, March 1983, pages 110 - 118, XP002972975
- MANDEVILLE R. ET AL.: 'Neutralization of Pseudotypes of Vesicular Stomatitis Virus by Sera from Avian Ret rovirus-Infected Hosts' INTERNATIONAL JOURNAL OF CANCER vol. 23, no. 3, 15 March 1979, pages 415 - 423, XP002972968

## Description

### Field of Invention

The present invention relates generally to the field of viral vectors having an altered host range. More specifically, the present invention relates to vector particles containing viral envelope with the foreign membrane-associated protein VSV G and the production of these particles from stable cell lines.

### Government Interest in Invention

This invention was made with government support under U.S. Public Health Service grant HD20034 from the National Institutes of Health. The government has certain rights in the invention.

### Background of the Invention

The assembly of enveloped animal viruses is characterized by selective inclusion of the viral genome and accessory viral proteins into a budding viral particle. Although the mechanisms for selective encapsidation or packaging are not well characterized, it has been postulated that the recognition of viral envelope proteins within the plasma membrane by the viral nucleocapsids represents one probable control point for packaging specificity.

Using internal image anti-idiotype antibodies, Vaux et al. (Nature (London) 336:36-42 (1988)) have shown that the nucleocapsid of Semliki Forest virus (SFV) contains a specific receptor for the cytoplasmic tail of the virion E2 spike glycoprotein. Vaux et al. suggested that a specific receptor-ligand-like interaction between the two is likely to be critical in the organization of the budding of SFV and related viruses from infected cells.

In apparent contrast to the high degree of specificity of SFV for the E2 protein, is the well-known phenomenon of "pseudotype" formation, in which mixed infection of a cell by one virus and retroviruses results in the production of progeny virions bearing the genome of one of the viruses encapsidated by the envelope proteins of the other. These phenotypically mixed viruses form plaques on appropriate indicator cells and can be neutralized by sera raised against the specific envelope protein. One virus known to participate in pseudotype formation is vesicular stomatitis virus (VSV), a member of the rhabdovirus family.

The mechanism for the inclusion of the envelope protein of one virus into the virions of an unrelated virus is uncertain. Sequence comparison of VSV G protein and retrovirus envelope proteins reveals no significant sequence similarity among these proteins. Heretofore, it has also been difficult to determine whether G protein alone in the absence of other VSV-encoded proteins can participate in pseudotype formation. Pseudotypes do not form between VSV and alphaviruses such as SFV even though pseudotypes can form between two alphaviruses or between alphaviruses and related flaviviruses such as Japanese encephalitis virus.

In some cases, phenotypic mixing is unilateral, as in the case of VSV with fowl plaque virus (FPV) or VSV with Sindbis virus. The pseudotype virus particle VSV(FPV) containing the VSV genome encapsidated by the envelope protein of FPV and the pseudotype virus particle VSV(Sindbis) have been demonstrated, but the reverse pseudotypes, FPV(VSV) and Sindbis(VSV), containing FPV or Sindbis virus genome with the VSV G protein, have not been detected.

Mixed infection of cells with retroviruses and VSV usually results in the formation of pseudotypes with much lower titers than that of VSV generated from the same cells. It is not clear whether this is due to the specificity of the interaction between the retroviral nucleocapsid and the G protein or due to other factors.

Retroviral vectors have been used to transfer genes efficiently by exploiting the viral infectious process. Foreign genes cloned into the retroviral genome can be delivered efficiently to cells susceptible to infection by the retrovirus. Through other genetic manipulations, the replicative capacity of the retroviral genome can be destroyed. The vectors introduce new genetic material to a cell but are unable to replicate. A helper virus or a packaging system can be used to permit vector particle assembly and egress. As used herein, the term "vector particle" refers to viral-like particles that are capable of introducing nucleic acid into a cell through a viral-like entry mechanism. Such vector particles, can under certain circumstances, mediate the transfer of genes into the cells they infect.

It is possible to alter the range of cells that these vectors can infect by including an envelope gene from another closely related virus. Miller et al. (Mol. Cell. Biol, 5:431-437 (1985)) constructed a MoMLV-derived vector to introduce a selectable marker, dihydrofolate reductase, into susceptible cells, and included the envelope region from the related amphotropic retrovirus 4070A to broaden the host range of the vector.

As stated above, it is not clear what signals are required to direct the functional assembly of the vector particle, nor is it known what factors permit the nucleocapsid and the membrane-associated protein to interact and complete packaging. Accordingly, heretofore, alterations in the host range have not been effected by including heterologous membrane-associated proteins within a vector particle. By "heterologous membrane-associated protein" it is meant a membrane-associated protein having at least one origin other than a virus of the same viral family as the origin of the nucleocapsid protein of the vector particle. As used herein, viral "family" shall be used to refer to the taxonomic rank of family, as assigned by the International Committee on Taxonomy of Viruses.

The present invention provides a mammalian packaging cell containing nucleotide sequences encoding gag, pol, and a heterologous chimeric membrane-associated protein, which determines a host range the chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G and wherein the exterior receptor binding domain is selected from any ligand/receptor to determine a host range which is different from that of the virus from which the nucleotide sequence encoding gag and pol derives.

In another aspect, the present invention provides a recombinant vector particle comprising gag and pol retroviral proteins and a nucleic acid sequence associatd with the vector particle; and an envelope comprising a heterologous chimeric membrane-associated protein, which determines a host range the chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G and wherein the exterior receptor binding domain is selected from any ligand/receptor to determine a host range which is different from that of the virus from which gag and pol derive.

The functional nucleic acia sequence of the vector particles of the present invention preferably comprises a long terminal repeat from a retrovirus, and also encodes a gene having an origin other than from the first virus, the gene being expressible into polypeptide, such as a selectable marker. A preferred selectable marker is a neomycin resistance gene. The gene encoding polypeptide can be expressed from the long terminal repeat, or an internal promotes which can direct transcription, and in a preferred embodiment from a tissue-specific promoter.

In another aspect, the present invention provides a method for producing a recombinant enveloped vector particle form a mammalian packaging cell, the recombinant virion comprising a chimeric membrane-associated protein, the method comprising the steps of: introducing a retroviral vector into a mammalian packaging cell, the cell stably expressing gag and pol, and expressing a heterologous chimeric membrane-associated protein which determines a host range, the chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G; and wherein the exterior receptor binding domain is selected from any ligand/receptor to determine a host range which is different from that of the virus from which gag and pol derive; culturing the mammalian packaging cell; and isolation a recombinant enveloped vector particle produced by the cell.

In still another method the invention relates to a method of producing an enveloped vector particle from a mammalian packaging cell line, the method comprising the steps of: introducing a retroviral vector into a stable mammalian packaging cell containing a first nucleotide sequence encoding gag an pol, and a second nucleotide sequence encoding VSV G which determines a host range being different from that of the retrovirus from which said first nucleotide sequence derives wherein said second nucleotide sequence is operably linked to an inducible promoter; culturing the cell. under conditions for induction of expression of VSV G from said inducible promoter; and isolating vector particles produced by the cell.

The stably transfected functional nucleic acid preferably encodes the functions for the production of nucleocapsid protein. The membrane-associated protein can alternatively be expressed from an inducible promoter, for example. Such stable cell lines allow the production of vector particles with essentially any desired functional nucleic acid. The desired functional nucleic acid can be introduced by, for example, transfection into these stable cell lines that express nucleocapsid and a membrane-associated protein and thereby result in the packaging of the desired functional nucleic acid with concomitant production of vector particles.

The present invention may be used in a method of introducing foreign nucleic acid into a cell, comprising: creating a vector particle having a functional nucleic acid sequence, a nucleocapsid protein derived from a first virus and a heterologous membrane-associated protein, the membrane-associated protein defining a host range including the cell; and infecting the cell with the vector particle. This creating step preferably comprises co-transfection with a helper virus or a functional part of a helper virus genome. In certain aspects of this invention, the membrane-associated protein is a chimeric protein including an exterior receptor binding domain and a membrane-associated domain, and the exterior receptor binding domaim is derived from a different origin than the membrane-associated domain which derives from VSV G protein. The chimeric protein is preferably derived from two origins, with no more than one of the two origins being retroviral. In a preferred embodiment the exterior receptor binding domain is from VSV G protein, and the first virus is a retrovirus, such as MoMLV, having a host range not including human cells or a subset of human cells.

The recombinant vector particles of the present invention may be used in a method for introducing nucleic acid into cells which can be part of a living organism, comprising infecting the cells with vector particles having the nucleic acid within a nucleocapsid and a lipid envelope, wherein tne nucleocapsid has an origin from a first virus, the envelope has an associated heterologous membrane-associated protein which determines a host range including the cells, and wherein the nucleic acid is transcribable into further nucleic acid. In certain preferred forms of the method, the method also includes transcribing the nucleic acid into complementary nucleic acid. In a preferred form of the method, the nucleic acid encodes a polypeptide, and the method includes expressing the polypeptide through translation of the complementary nucleic acid.

The nucleic acid used with this method can encode a therapeutic agent. In one form, the method can be used as therapy for a genetic defect in which a polypeptide is not expressed in sufficient quantities in active form, wherein the polypeptide encoded by the nucleic acid is an active form of the polypeptide not expressed in sufficient quantities, and wherein the cells infected with the vector particles are cells from the mammal.

Preferably, the method includes identifying target cells in the organism in which it is desired to introduce the nucleic acid, wherein the membrane-associated protein recognizes a receptor on the target cells, and also includes integrating the nucleic acid into the genome of the cells.

Further objects, features and other advantages of the present invention will become apparent from the ensuing detailed description, considered together with the appended drawings.

### Brief Description of the Figures

Figure 1 is a schematic representation, not drawn to scale, of retroviral vectors and packaging constructs pLRNL, pLARNL, pLGRNL, pSVGP, and pSAM for the creation of retroviral vector particles.

Figure 2 shows a Southern blot analysis of LGRNL infected cells using neomycin resistance gene as probe.

Figure 3 shows immunoprecipitates separated by SDS-PAGE and visualized by fluorography. Lanes 1 and 2 show the polyclonal cell line immunoprecipitated with anti-VSV G and normal rabbit serum, respectively. Lane 3 is mock infected MDCK cells immunoprecipitated with anti-VSV G antibody.

Figure 4 shows immunoprecipitates of selected cells pooled, grown for 1 week, and then reisolated by FACS sorting. Lane 1 shows the extracts of the twice-sorted cells immunoprecipitated with the anti-VSV-G antibody and lanes 2, 3 and 4 correspond to lanes 3, 2 and 1 of Figure 3, respectively.

### Detailed Description of the Invention

We have discovered that VSV-G membrane-associated protein can be incorporated into retroviral vector particles to provide an altered host range throughout a broad spectrum of cells.

As a model system, we incorporated the VSV G membrane-associated protein into Moloney murine leukemia virus (MoMLV)-based vector particles to generate a retroviral vector particle having a nucleocapsid derived from MLV and the membrane associated protein VSV G within its envelope. Such vector particles shall be referred to herein by the designation MLV[VSV G]. This designation shall be used herein generally to refer to vector particles having a nucleocapsid from one viral origin and a membrane-associated protein from another origin, with the non-bracketed portion of the designation referring to the capsid origin and the bracketed portion referring to the membrane-associated protein within the envelope of the vector particle.

For production of vector particles, the nucleic acid of the vector particles of the present invention can be used to transfect a suitable cell line. The vector particles are released into the supernatant from the transfected cells and used to infect a desired cell type. The nucleic acid used for transfection can either be in isolated form or can be already packaged into vector particles. In some instances, a helper virus is required to facilitate virion assembly. Thus, for example, the genes encoding the gag and pol genes can be incorporated into the nucleic acid of a helper virus and functional sequences from another virus can be incorporated into the nucleic acid which is packaged into the vector particles.

The gene encoding a membrane-associated envelope protein can be incorporated within the nucleic acid of either the vector particle or of the helper virus. Alternatively, the gene for this envelope protein could be expressed from a third fragment of nucleic acid or from the genome of the producer cell. In a preferred form of the present invention, the nucleic acid within the vector particle is integrated into the cellular genome of the cell infected by the vector particle, the envelope gene is located on a different fragment of nucleic acid than the nucleic acid that is vector particle genome. Thus, in this preferred embodiment, the membrane-associated protein will not be produced by the vector particle infected cells containing the integrated nucleic acid from the vector particle.

As will be described hereinbelow, we have discovered that the VSV G protein alone is sufficient to interact with the nucleocapsid of MoMLV in the formation of MLV[VSV G] vector particles. The process of incorporation of the VSV G protein into the vector particles is efficient and results in the production of infectious vector particles with titers comparable to that of whole retroviruses. Thus, we believe that other heterologous membrane-associated proteins can also be efficiently incorporated into the envelopes of enveloped viruses.

MoMLV is a murine retrovirus which has poor infectivity outside of mouse cells. When this ecotropic virus is adapted to produce retroviral vector particles carrying, for example, the N2 vector genome, this vector will infect only mouse cells at appreciable efficiencies. The related amphotropic N2 virus will infect cells from human, mouse and other organisms. This difference is attributable to the substitution of the amphotropic envelope protein for the ecotropic envelope protein. Both types of viral vector particles have essentially identical nucleocapsids derived from MoMLV. However, neither ecotropic N2 nor amphotropic N2 virus will infect hamster cells. As shown in the Examples provided hereinbelow, we have discovered that hamster cells can be infected by MLV[VSV G] vector particles and that addition of anti-VSV serum to preparations of these viral particles completely abolished their infectivity. Thus, we have shown that the presence of VSV G protein in the vector particles results in vectors having a host range derived from VSV, the origin of the membrane-associated protein incorporated within their envelope.

In order to determine if vector particles containing heterologous membrane-associated protein could be efficiently assembled, we tested whether the VSV G protein could be assembled with the nucleocapsid of MoMLV. We first introduced the neomycin phosphotranferase gene (Neo) which provides neomycin resistance, as a selectable marker for the vector particles into the amphotropic packaging cell line PA317. The resultant vector particles were added to cells incapable of supporting amphotropic MLV infection. Baby Hamster Kidney (BHK) cells lack amphotropic cell surface receptors and are, therefore, not susceptible to infection with amphotropic MLV-based retroviruses, as shown below in the experiments of Example 1.

In initial experiments, we confirmed that this cell line is indeed refractory to infection by amphotropic MLV retrovirus. The next set of initial experiments is shown in Example 1. In these experiments, we confirmed that amphotropic N2 virus containing the Neo gene will grow in rat 208F fibroblasts, but not in BHK cells.

### EXAMPLE 1

### Growth of N2 Virus in Rat 208F Fibroblasts

Amphotropic N2 virus containing the Neo gene inserted between the long terminal repeats (LTRs) of MoMLV was prepared from the producer cell line PA317/N2 and titered both on BHK cells and rat 208F fibroblasts, a cell line that is susceptible to MoMLV retrovirus infection. We observed a 10⁵-fold decrease in Neo-resistant (Neo^{r}) colony forming units (CFU) in BHK cells compared with that in rat 208F cells; thus, the BHK cell we used failed to support infection by N2 virus containing the amphotropic retroviral envelope protein.

With the initial studies completed, we produced enveloped vector particles containing heterologous membrane-associated proteins. As an example of such production, Example 2 is provided to show the production of amphotropic N2 MLV[VSV G] vector particles using an MLV-based retroviral vector encoding the VSV G protein. We assayed the MLV[VSV G] vector particles on BHK cells to determine if the host range of these vector particles had been altered relative to amphotropic MLV.

### EXAMPLE 2

### Generation of MLV[VSV G] Vector particles

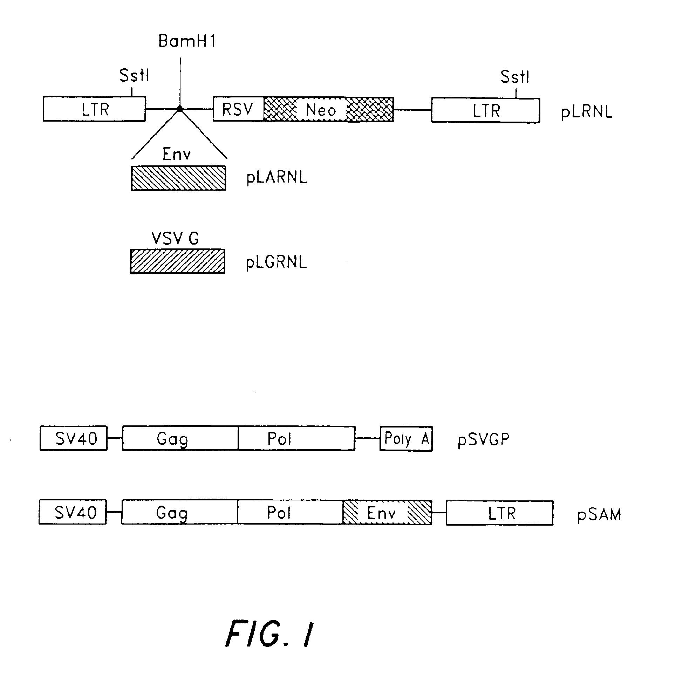

To produce MLV[VSV G] vector particles, we used MoMLV-based retroviral vectors as illustrated in Figure 1, which is not drawn to scale. In pLRNL, the Neo^{r} gene expressed from the Rous Sarcoma Virus (RSV) promoter was inserted between the LTRs of MoMLV. The MoMLV-based retroviral vector pLRNL contains the Neo-resistance (Neo) gene under control of the promoter of RSV. This vector is described in Li et al., Virology 171:331-341 (1989), the disclosure of which is hereby incorporated by reference. A single BamHl site is immediately upstream of the RSV promoter. Into this BamHl site, a 1.7-kilobase pair (kb) BamHl fragment containing the entire coding region of VSV G gene was inserted, giving rise to the construct pLGRNL. This VSV G gene is described in Rose et al., Cell 30:753-762 (1982) and Rose et al., J. Virol., 39:519-528 (1981), the disclosures of which are hereby incorporated by reference. The plasmid pSAM, also shown in Figure 1, containing the gag region of MoMLV, a hybrid pol region between MoMLV and amphotropic virus 4070A and the env region of 4070A has been described in Miller et al., Science, 225:630-632, the disclosure of which is hereby incorporated by reference.

HPRT-deficient 208F cells were derived from Fischer rat cells by selection in 6-thioguanine, as described in Quade, K. Virology, 98:461-465 (1979), the disclosure of which is hereby incorporated by reference. Thymidine kinase (Tk)-deficient BHK cells were derived from BHK21 cells by selection with 5-bromodeoxyuridine, as described in Littlefield et al., Nature 211:250-252 (1966), the disclosure of which is hereby incorporated by reference. These BHK cells and 208F fibroblasts were grown in Dulbecco-modified Eagle medium (DME) with high glucose supplemented with 10% fetal calf serum (FCS).

To generate infectious vector particles, either pLGRNL or pLRNL was co-transfected into BHK cells with the helper vector pSAM expressing MoMLV gag protein, a polymerase gene and amphotropic envelope protein from the SV40 early promoter. Supernatants from transfected BHK cells were collected at 48 hours post-transfection and used to infect susceptible 208F cells and resistant BHK cells. To titer virus, cells were infected overnight with filtered supernatants in the presence of 4 mg/ml polybrene. Infected cells were selected in medium containing 400 mg/ml G418 and colonies were scored about 14 days after infection. The results of the experiment of this example are shown in Table 1.

**TABLE 1**

| Transient vector particle production from BHK cells following cotransfection by pSAM with either pLRNL or pLGNRL | | |
|---|---|---|
| Vector Particle | Cell infected | Titer (CFU/ml) |
| LRNL | 208F | 480 |
| | BHK | <10 |
| | | |
| LGRNL | 208F | 380 |
| | BHK | 260 |

It can be seen from Table 1 that vector particles derived from pLRNL lacking VSV G protein were able to infect 208F cells efficiently but failed to infect BHK cells. In contrast, vector particles derived from pLGRNL, containing the VSV G protein, could infect not only 208F cells, but also BHK cells, as indicated by the appearance of Neo^{r} colonies. Thus, the host range of the MLV[VSV G] vector particles had been altered relative to amphotropic MLV.

Accordingly, we believe that the VSV G protein produced by the pLGRNL-transfected BHK cells of Example 2 was incorporated into at least some of the retroviral vector particles, producing MLV[VSV G] vector particles capable of infecting hamster cells. Since the only VSV protein encoded by pLGRNL is the VSV G protein, the results of Example 2 shown in Table 1 also indicate that G protein alone, without the participation of other VSV-encoded proteins, is sufficient for the formation of MLV[VSV G] vector particles. Accordingly, Example 2 shows that infectious vector particles could be assembled having nucleocapsid from one virus and a heterologous membrane-associated protein.

As stated above in the background of the invention, mixed infection of VSV and retroviruses resulted in a lower titer of vector particles than what is obtained from VSV infection alone. Rather than being the result of inefficient incorporation into virions caused by poor specificity between the G protein and the retroviral nucleocapsid, we believe that this lower titer is due to the progressive inhibition of cellular protein synthesis, including those proteins encoded by the retroviruses, as a result of VSV G protein toxicity.

Thus, in order to determine whether the presence of membrane-associated protein from the same family of viruses as the origin of the nucleocapsid is necessary for the formation of vector particles, we conducted experiments to form NLV[VSV G] vector particles without any envelope protein having an origin from the same family as MLV. An example of such an experiment is shown in Example 3.

### EXAMPLE 3

### Formation of MLV[VSV G] Vector particles without Env Gene Product

For this experiment, we produced two additional vectors shown in Figure 1, pLARNL and pSVGP. pLARNL is produced from pLRNL in a manner similar to the production of pLGRNL, however, a 2.7-kb Xbal fragment containing the envelope (env) gene of amphotropic retrovirus 4070A was inserted into the BamHl site in pLRNL. pSVGP was constructed by inserting a 5.8 kb HindIII-Scal fragment containing the SV40 early promoter and the gag and the pol regions from pSAM into the mammalian expression vector pcD, described in Okayama et al., Mol. Cell. Biol. 3:280-289 (1983), the disclosure of which is hereby incorporated by reference. "Poly A" in Figure 1 indicates the polyadenylation signal derived from SV40.

In this example, we co-transfected pLRNL, pLARNL or pLGRNL with pSVGP into BHK cells. To generate vector particles, 10 µg of the vector DNA together with 10 µg pSVGP were co-transfected into cells by using the calcium phosphate precipitation procedure of Graham et al, Virology 52:456-457, the disclosure of which is hereby incorporated by reference. Culture medium was collected 36h after transfection and filtered through a 0.45-mm filter.

The construct pSVGP contains gag and pol genes expressed from the SV40 early promoter, but the MoMLV Env gene is absent. The vector particles expressing the Neo^{r} marker (pLARNL or pLGRNL) were titered separately on 208F and BHK cells, as described above. The titers obtained are shown in Table 2.

**TABLE 2**

| Transient vector particle production from BHK cells following cotransfection by pSVGP with pLRNL, pLARNL or pLGRNL | | | |
|---|---|---|---|
| Experiment No. | Vector Particle | Cell infected | Titer (CFU/ml) |
| 1 | LRNL | 208F | <10 |
| | | BHK | <10 |
| | | | |
| | LARNL | 208F | 280 |
| | | BHK | <10 |
| | | | |
| | LGRNL | 208F | 440 |
| | | BHK | 680 |
| | | | |
| 2 | LRNL | 208F | <10 |
| | | BHK | <10 |
| | | | |
| | LARNL | 208F | 260 |
| | | BHK | <10 |
| | | | |
| | LGRNL | 208F | 480 |
| | | BHK | 720 |

It can be seen from the results of Table 2 that no infectious vector particles were detected from pLRNL and pSVGP co-transfected cells since the MoMLV env gene was absent in both plasmids. 208F cells co-transfected with pLARNL, containing the amphotropic MLV Env gene, and pSVGP produced a Neo^{r} titer of 200-300 CFU/ml, but this preparation failed to infect BHK cells. However, both 208F and BHK cells were infected with similar efficiencies by pLGRNL derived vector particles, containing the VSV G gene, cotransfected with pSVGP. Since the LGRNL vector particles were generated in the complete absence of MoMLV env protein, the results of Example 3 shown in Table 2 lead us to the conclusion that a heterologous membrane-associated protein can be assembled into the vector particles without the participation of any envelope protein having an origin from the same family of viruses as the origin of the nucleocapsid. Since G protein is also the sole VSV-encoded protein used in this Example, this confirms that no VSV gene products other than G protein are required for the formation MLV[VSV G] vector particles. Thus, we believe that no additional gene products are generally necessary for the introduction of foreign membrane-associated proteins into vector particles.

We obtained similar titers of Neo-resistance when transiently generated LGRNL and LARNL vector particles were assayed on 208F cells. Since expression of both the retrovirus env gene and the VSV G gene is regulated by the same MoMLV LTR in these constructs, we believe the amounts of transiently produced proteins in the transfected BHK cells are similar. However, we can not exclude the possibility that the stability of the two proteins may be different. In any event, we believe that other membrane-associated proteins can be similarly expressed using these and similar constructs.

Thus, we believe that the interaction between nucleocapsids and the heterologous membrane-associated protein can be at least as efficient or more efficient than with heterologous membrane-associated protein. In the case of MLV[VSV G] vector particles, we observed greater efficiency of infection of 208F cells by MLV[VSV G] vector particles at lower titers than with vector particles having both nucleocapsid and membrane-associated protein derived from MLV. The receptors for murine retroviruses, such as MLV, have been shown to be cell surface proteins, whereas there is some evidence that a membrane phospholipid is the receptor for VSV, possibly accounting for the wide host range of VSV. Therefore, we believe that our observed Neo^{r} titers of MLV[VSV G] vector particles reflects increased cellular susceptibility to infection with MLV[VSV G] vector particles than with env-containing MoMLV vector particles.

To ascertain if the specificity of infection with vector particles, such as MLV[VSV G], is determined by the membrane-associated protein, the vector particles can be treated with neutralizing antiserum having specificity to the membrane-associated protein. If the specificity is determined by the membrane-associated protein, such neutralizing antiserum would prevent infection by the neutralized particles. Thus, we performed the experiment of Example 4 in order to determine if vector particles having a heterologous membrane-associated protein provided a specificity of infection determined by the membrane-associated protein. In Example 4, anti-VSV serum was used to neutralize the infectivity of the MLV[VSV G] vector particles.

### EXAMPLE 4

### Neutralization of MLV[VSV G] Vector particles

Vector particles, generated transiently by co-transfection of pLARNL or pLGRNL with pSVGP, were incubated at 37°C for 45 min either with normal rabbit serum or with various dilutions (1:500, 1:100 and 1:20) of rabbit anti-VSV serum purchased from Lee Biomolecular Research Laboratories, Inc. Supernatants were harvested 48 h after cotransfection of pSVGP with pLARNL or pLGRNL into BHK cells. The vector particles were quantitated by assaying for the formation of Neo^{r} colonies on 208F cells and BHK cells, as described above. The results are shown in Table 3.

**TABLE 3**

| Neutralization of MLV[VSV G] vector particles with anti-VSV antibody | | | | | | |
|---|---|---|---|---|---|---|
| Vector | Normal rabbit | Anti-VSV serum | | | Titer (CFU/ml) in | |
| | | 1:500 | 1:100 | 1:20 | 208F | BHK |
| LARNL | -- | -- | -- | -- | 240 | <10 |
| | + | -- | -- | -- | 230 | <10 |
| | -- | -- | + | -- | 230 | <10 |
| | -- | -- | -- | + | 220 | <10 |
| | | | | | | |
| LGRNL | -- | -- | -- | -- | 620 | 880 |
| | + | -- | -- | -- | 600 | 880 |
| | -- | + | -- | -- | 110 | 150 |
| | -- | -- | + | -- | <10 | <10 |
| | -- | -- | -- | + | <10 | <10 |

It can be seen from the results shown in Table 3 that anti-VSV serum at a dilution of 1:100 markedly reduced the infectivity of LGRNL vector particles on both cell types whereas the titer of LARNL on 208F cells was not affected by exposure to VSV antiserum. This observation further supports our belief that VSV G protein is assembled into the vector particles of LGRNL.

As was shown by the results of Example 3 detailed in Table 2, incorporation of VSV G protein into vector particles was efficient, since vector particles transiently generated by cotransfecting pLGRNL or pLARNL with pSVGP gave similar Neo^{r} titers on 208F cells. We next determined whether the process of incorporating membrane-associated proteins, such as VSV G protein, into particle envelopes is equally efficient in the presence of the native particle envelope protein. This determination was done by co-transfecting pLGRNL with pSAM into BHK cells and analyzing the proportions of pure vector particles and phenotypically mixed particles by anti-VSV antibody-directed, complement-mediated lysis. These experiments are shown in Example 5.

### EXAMPLE 5

### Complement-Mediated Lysis of MLV[VSV G] Vector particles

Vector particles containing cell supernatant in a final volume of 200 µl of DMB-10% FCS containing various amounts of specific antisera, complement or equivalent volumes of normal serum were heated at 37°C for 45 minutes. The mixture was then added to culture media in the presence of 4 µg/ml polybrene. Infected cells were selected in G418-containing medium as described above. Viral supernatants were harvested 48 h after cotransfection of pSAM with pLARNL or pLGRNL into BHK cells. The supernatants were treated with either 10 µl rabbit serum or 10 µl anti-VSV serum at 1:20 dilution with or without 10 µl rabbit complement. All reactions were done in a final volume of 200 µl, heated at 37°C for 45 min. The results are shown in Table 4.

**TABLE 4**

| Antibody complement-mediated lysis of MLV[VSV G] vector particles | | | | | |
|---|---|---|---|---|---|
| Vector particle | Normal rabbit | Anti-VSV | Rabbit | Titer (CFU/ml) in | |
| | serum | serum | complement | 208F | BHK |
| LARNL | -- | -- | -- | 480 | <10 |
| | + | -- | -- | 460 | <10 |
| | -- | -- | + | 410 | <10 |
| | -- | + | + | 400 | <10 |
| | | | | | |
| LGRNL | -- | -- | -- | 380 | 260 |
| | + | -- | -- | 380 | 250 |
| | -- | -- | + | 320 | 230 |
| | -- | + | -- | 270 | <10 |
| | -- | + | + | 90 | <10 |

It can be seen from Table 4 that without any treatment, transiently generated LGRNL vector particles gave a Neo^{r} titer of 380 CFU/ml on 208F cells and 260 CFU/ml on BHK cells. Addition of normal rabbit serum to the same vector particle preparation had no effect on titer. In contrast, the titer dropped to 270 CFU/ml on 208F cells and to background levels on BHK cells when the vector particles were pre-treated with a 1:20 dilution of the anti-VSV antibody. This result indicates a titer of LGRNL vector particles containing only VSV G protein in this preparation of approximately 110 CFU/ml on 208F cells. To determine the fraction of vector particles containing only the retroviral envelope protein and the fraction of vector particles containing both the retroviral envelope protein and the VSV G protein in this preparation, we treated the vector particle preparation with a combination of rabbit complement and the anti-VSV antibody.

The potentiating effect of complement-mediated lysis has been shown previously by others to eliminate MLV[VSV G] vector particles efficiently. Addition of rabbit complement alone to the vector particle preparation slightly reduced the Neo^{r} titer to 320 CFU/ml on 208F cells and 230 CFU/ml on BHK cells (see Table 4), probably due to the non-specific interaction of fetal calf serum in the media with rabbit complement. Vector particles pre-treated with a 1:20 dilution of the anti-VSV antibody together with rabbit complement gave a Neo^{r} titer of 90 CFU/ml on 208F cells. Thus, we believe that this non-neutralized population of Neo^{r} vector particles contains only the retroviral envelope protein. This interpretation is consistent with the results also shown in Table 4, in which similar treatments of LARNL vector particles containing only retroviral envelope protein have little effect on its Neo^{r} titer when measured on 208F cells. Thus, it is likely that VSV G protein and other membrane-associated proteins can be efficiently incorporated into vector particles even in the presence of native envelope proteins.

Retroviral DNA integrates into host chromosomes in a fashion that maintains the linear organization of the viral genome in sequences called proviral sequences. To establish that cell clones resulting from infection with enveloped vector particles having a heterologous membrane-associated protein contained proviral sequences that maintain the linear organization of the vector particle nucleic acid, we performed Southern blot analysis of LGRNL-infected clones. These experiments are shown in Example 6.

### EXAMPLE 6

### Southern Blot Analysis of LGRNL Infected Cells

Genomic DNA was prepared as described by Maniatis et al., "Molecular Cloning, A Laboratory Manual," Cold Spring Harbor Laboratory, Cold Spring Harbor, NY (1982), the disclosure of which is hereby incorporated by reference. DNA samples were digested with appropriate restriction enzymes, electrophoresed on 0.8% agarose gels and transferred to nylon membranes. A ³²P-labeled DNA probe containing the complete neomycin phosphotransferase gene prepared by priming with random deoxy hexanucleotides (Amersham) was used as a hybridization probe with the filters. Filters were washed with 0.1 X SSC-0.5% SDS at 53°C several times and subjected to autoradiography.

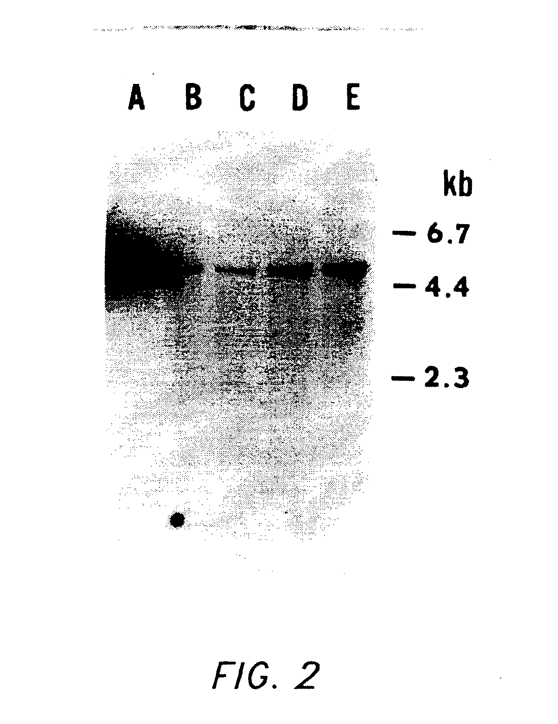

Genomic DNA was isolated from two different Neo^{r} clones (lanes B and C) and two different Neo^{r} BHK clones (lanes D and E) that were infected with LGRNL vector particles. DNA (10 µg) was cut with Sstl, run on a 1% agarose gel, transferred to a nylon-type filter and probed with a Neo gene-specific probe. Five nanograms of pLGRNL plasmid DNA digested with Sstl was loaded in lane A to serve as a marker. The resulting Southern blot is shown in Figure 2.

Sst I cleaves only once in each LTR of LGRNL and is therefore expected to yield a 5-kb fragment (see Figure 1). As seen in lanes B through E of Figure 2, when the chromosomal DNA from the two BHK clones and two 208F clones was cleaved with Sst I and hybridized to a probe containing the Neo gene, a fragment of about 5 kb was detected. The size of this fragment was identical to that of the Sst I-cleaved pLGRNL plasmid DNA seen in lane A. Thus, the results of Example 6 show that the LGRNL proviral DNA appears to have an uninterrupted and un-rearranged organization in the infected cells. This result is the same as that seen for wild-type retroviral infection. Thus, we believe that other recombinant vector particles will similarly form uninterrupted and un-rearranged proviral DNA sequences.

In order to illustrate that such recombinant vector particles obtain the host range of the membrane-associated protein present in their envelope, we infected a variety of cell lines with vector particles derived from pLGRNL and pLARNL. These studies are shown in Example 7.

### EXAMPLE 7

### DETERMINATION OF HOST RANGE OF MLV[VSV G]

HeLa cells were obtained from the American Type Culture Collection. LNSV cells were derived from SV40-transformed HPRT-deficient Lesch-Nyhan cells (described in Jolly et al., Mol. Cell. Biol., 6:1141-1147 (1986), the disclosure of which is hereby incorporated by reference). Rat 208F cells and BHK cells were as previously described. Supernatants were harvested 48 h after cotransfection of pSVGP with pLARNL or pLGRNL into BHK cells. Equal volumes of each supernatant was then applied to the four different cell types and Neo^{r} titers were determined two weeks after G418 selection. The experiment has been repeated three times and the result of one such experiment is shown in Table 5.

**TABLE 5**

| Relative susceptibility of different cell lines to MLV[VSV G] vector particles | | |
|---|---|---|
| | Titer (CFU/ml)^{a} of | |
| Infected cell | LARNL | LGRNL |
| 208F (rat fibroblast) | 250 | 450 |
| BHK (hamster kidney) | <10 | 680 |
| LNSV (human fibroblast) | 16 | 180 |
| Hela (human carcinoma) | 12 | 30 |

As described above, the retroviral env gene of LARNL used in this study is derived from the env regions of amphotropic virus 4070A. The envelope glycoprotein encoded by this gene can bind to cell surface receptors present on cells from a wide range of mammalian species, including human cells. However, several human lines were relatively refractory to amphotropic viral infection compared to murine cells. As shown in Table 5, the Neo^{r} titers of LARNL in human lines such as HeLa cells or LNSV fibroblasts are about 20 fold lower relative to that in rat 208F cells. As also shown in Table 5, LGRNL vector particles infected HeLa cells with an efficiency slightly greater than LARNL vector particles; however, they infected LNSV fibroblasts much more efficiently than did LARNL vector particles.

Due to the excellent efficiency of infection of the MLV[VSV G] vector particles derived from pLGRNL on LNSV cells, we believe that the major block to infection of LNSV cells with retrovirus is due to inefficient interaction of cell surface receptors with retroviral envelope protein. The inclusion within the retrovirus of membrane associated protein known to interact with cell surface receptors appears to overcome this block. Thus, we believe that we have discovered a general method for altering the host range of enveloped vector particles by including a heterologous membrane-associated protein within the envelope of vector particles derived therefrom.

We do not believe the relatively less efficient results seen with infection of HeLa cells by the MLV[VSV G] vector particles of LGRNL are due to poor interaction with the HeLa cell surface receptors. Rather, since VSV infects HeLa cells efficiently, we believe the less efficient results observed above are probably due to post-penetration events.

Currently, retroviral vectors provide the most efficient method for introducing new genetic information into mammalian cells. Since vector particles containing amphotropic envelope protein can infect human cells, they represent a potentially useful tool for the treatment of genetic disorders in human. However, as shown in Table 5, some human cells can be relatively resistant to amphotropic retrovirus infection. The presence of relatively few amphotropic retrovirus receptors, inefficient reverse transcription in the cytoplasm or accelerated degradation of unintegrated viral DNA in human cells can each account for the inefficient infection of human cells by the retroviral vectors.

Our discovery provides a method of increasing the efficiency of retroviral infection of human and other cells beyond the limited range provided by membrane-associated proteins derived from the same family of viruses. This discovery is that incorporating membrane-associated proteins into the retrovirus can overcome at least some of the potential blocks for infection by altering the host range of the retroviral vectors, thus facilitating more efficient transfer of foreign genes into these cells. This system uses helper virus to create an infective unit. This unit contains elements of a retrovirus that permit integration into the host genome, regulatory elements to control expression of a foreign gene, a selectable cell marker and/or other foreign genes. This unit is designed to target a given group of cells. The breadth of infectivity being defined by the ability of the engineered protein to bind a range of cell types. These component genes can be introduced into the resulting vectors through any manner known to those of skill in the art.

It is envisioned that other viruses could be used in this system. Long terminal repeats from other retroviruses could be used from a group consisting of but not limited to RSV, HIV, Avian Leukemia Virus and other murine leukemia viruses. The capsid protein could also be derived from these or other viruses.

The inventors have used in their model, an RSV promoter to control neomycin-resistance expression. Other markers include hygromycin-resistance and dihydrofolate reductase (methotrexate resistance). Similarly, other promoters could be used, including but not necessarily limited to the cytomegalovirus immediate early promoter, SV40. Promoters could be chosen so as to potently drive expression or to produce relatively weak expression, as desired.

Additionally, foreign genes for expression within a targeted cell population can be added to the vector, as will be described in more detail hereinbelow. Promoters with given tissue specificities could also be used in order to restrict expression of such foreign genes to a given cell type. For example, a pancreatic elastase promotor is specific for expression in pancreatic tissue. Another example would be the internal promoter of the human hepatitis B core gene which regulates gene expression in a liver-specific manner. Other tissue-specific promoters exist for brain and other tissues.

Membrane-associated proteins other than VSV G protein which are good candidates for providing altered host range when used in accordance with the present invention as described in the examples for VSV G protein include those proteins from other enveloped viruses that bind host receptors and facilitate infection. Our discovery allows for selective removal of any membrane-associated protein and use of this protein to create a nucleic acid delivery system that targets an altered range of cells. As one example, the gD gene from HSV (Herpes Simplex Virus) could be used in order to obtain a host range including the neural ganglia of human tissue. A large number of such viral and non-viral examples will be readily apparent to those of skill in the art.

Non-viral membrane-associated proteins could also be used to alter the host range of vector particles. We believe that proteins which interact as a ligand to a given cell receptor or cell surface moiety is a candidate to target a vector particle to a host range of cells having the given cell receptor or cell surface moiety. A suitable protein would contain binding regions which would serve to identify a host range for the vector particle. Depending on the ubiquity of the receptor for the protein in question, one could either target the viral vector to a vast range of human cells, to a subset of cells or to a single cell type. Thus, for example one could potentially target all human cells, all white blood cells, or only T-helper cells.

In one embodiment of this invention, such proteins are derived from viruses known to infect specific cell types. However, the present invention is not limited to the use of virally derived proteins. It is envisioned that any ligand/receptor duo could be exploited in this system. The requirements for an operable system being the identification of a ligand/receptor duo or, for therapy, at least an understanding of the breadth of cell types that could interact with the ligand. The utility of the system will depend on the availability of a receptor for a ligand and the affinity of a given receptor for that ligand. Thus, for example, CD4, known to interact with HIV gp120 on the surface of HIV infected cells, is a candidate to be used in a recombinant vector particle to deliver a foreign gene to those cells.

Example 8 is provided to illustrate the production of a vector which will target cells infected with HIV. The T-helper cell membrane-associated surface antigen, CD4 is known to be the major HIV cell surface receptor. The CD4 antigen has a ligand-receptor interaction with the HIV-produced protein, gp120. Thus, vector particles having CD4 associated with their viral envelope should target cells infected with HIV.

### EXAMPLE 8

### Vector Particle for Targeted HIV Therapy

Sequences encoding CD4 are first obtained from a T-cell line. Briefly, cells are grown in culture and lysed. Total RNA is purified from these cells using a cesium chloride gradient. The total RNA is washed and precipitated, and cDNA is synthesized therefrom using reverse transcriptase and oligo-dT primers. The resulting cDNA/RNA hybrids are used as template for Polymerase Chain Reaction (PCR) using appropriate primer pairs for CD4 that are complementary to both the 5' and 3' ends of the CD4 sequence, and include tails coding for Bam H1 restriction endonuclease sites. The PCR product is run on an agarose gel and the fragment of the expected size (approximately 1.2 kilobases) is cut and eluted from the gel. The eluted DNA is digested with Bam H1 and a promoter is added to the 5' end of this CD4 encoding sequence. The resulting sequence is cloned into the Bam H1 site in pLRNL.

Thus, Example 8 shows the production of a vector particle having a host range defined by a naturally occurring membrane-associated protein. When the ligand to be included within the viral envelope is not a naturally occurring membrane-associated protein, it is necessary to associate the ligand with the membrane. In order to accomplish this, the gene coding for the ligand can be combined with sequences coding for a membrane-associated domain. By "naturally occurring membrane associated protein", it is meant those proteins that in their native state exist in vivo in association with lipid membrane such as that found associated with a cell membrane or on a viral envelope.

In a preferred form of the present invention, this can be accomplished by recombining the gene coding for the ligand proximate of the membrane-binding domain for VSV G protein or other virally derived envelope protein that stably assembles with a given capsid protein. Thus, proteins, such as insulin, known to interact with receptors on muscle and adipose cells, potentially provide a vehicle for obtaining infectivity of cells having the appropriate insulin receptor by including the insulin gene with the VSV G sequences coding for its membrane-associated domain, or the membrane-associated sequences from another membrane-associated protein which forms a stable vector particle.

As stated above, we believe that the major block to infection of cells with enveloped vector particles is due to inefficient interaction of cell surface receptors with viral envelope protein. Thus, the exterior domain of these proteins could be linked to a membrane or interior domain to create a functional vector particle. The ligand-receptor interaction does not appear to be involved in the interaction between protein and nucleocapsid.

Although the nucleocapsid of Semliki Forest virus contains a specific receptor for the cytoplasmic tail of the viral E2 spike glycoprotein, this mechanism is not necessarily directly applicable to other enveloped viruses. Thus, for SFV and other alphaviruses, it is likely that E2 spike glycoprotein or a similar protein is required for efficient infection. Accordingly, if SFV or other alpha virus derived vector particles are to be used within the context of the present invention, it is necessary to include those portions of the E2 spike glycoprotein important for efficient interaction with the nucleocapsid.

Retroviruses, such as Rous sarcoma virus are able to produce vector particles using mutant forms of the envelope protein that lack a cytoplasmic tail. Thus, we believe that if an interaction between the envelope protein of retrovirus and its nucleocapsid is required to mediate the incorporation of the glycoprotein into the envelope of budding viral particles, such interaction occurs within or close to the lipid bilayer. Presumably, the interaction occurs within the hydrophobic membrane-associated domain of the envelope protein. Thus, we believe that for many systems, if any portion of the membrane-associated protein is important for vector particle assembly, this portion occurs within the membrane-associated domain or close to that domain.

Accordingly, we believe that forming recombinant proteins with membrane-associating domains from proteins having the ability to direct packaging, such as the VSV G protein, and at least the receptor binding portions of other ligands, whether membrane-associated or not, will permit vector particle targeting to desired cell types.

Thus, as one embodiment of the present invention, a cassette-type system is provided in which the portion of the gene coding for the membrane-associated domain from the VSV G protein or other membrane-associated protein remains as the constant portion of the cassette vector. Into this constant region is inserted portions from a desired ligand. These two domains form a recombinant protein which will interact with the nucleocapsid to provide for a structurally intact vector particle with efficient targeting capabilities for a desired cell type or types. It is important to maintain the reading frame for translation when inserting ligands into this cassette in order to obtain a full length, bifunctional recombinant protein chimera. A "protein chimera" is used to describe a product produced recombinantly by genetically linking regions of nucleic acid sequences from two or more proteins such that the encoded product, though one continuous protein, contains domains duplicating sequences of amino acids from those proteins. Consequently there is no need for using full length envelope protein or non-membrane bound protein, as long as sufficient ligand receptor interactions remain.

Thus, the present invention provides nucleic acid having functional sequences from a first virus and also sequences encoding a ligand identifying the host range of the vector particles containing the nucleic acid. By "functional" it is meant any sequences which are involved with typical wild-type replication or infection by that virus. Sequences other than those associated with typical wild-type replication or infection can also be included within the functional nucleic acids provided herein.

The nucleic acid can direct production of vector particle by itself, or can require co-transfection with a helper virus, as described above in connection with the use of LGRNL with pSVGP. With the use of these nucleic acid sequences, vector particles can be assembled that contain these host altering membrane-associated proteins, either as hybrid recombinant proteins or as expression products of a full length gene, to target a given particle to a given cell for gene delivery.

The envelope protein from a typical retrovirus, such as MoMLV, contains three domains: an exterior receptor binding domain, a membrane-associated domain and an interior (cytoplasmic) domain. Heretofore, it was thought that the cytoplasmic domain was required to interact with nucleocapsid protein for efficient particle packaging. Example 9 shows the results from an experiment that confirms that none of the retroviral envelope protein domains are required for efficient packaging of vector particles having a retrovirally derived nucleocapsid. In the experiment of Example 9, recombinant chimeric proteins having various retroviral domains were constructed and the packaging efficiency of the resulting vector particles assayed by the ability of the constructs to introduce VSV immunoreactivity into COS cells.

### EXAMPLE 9

### Packaging Efficiency of Vector Particles Having Retroviral/VSV G Chimeric Membrane-Associated Protein

Two chimeric membrane-associated protein gene constructs were prepared. The M2 construct contained VSV G exterior receptor binding domain fused to the membrane-associated and cytoplasmic domains of MLV envelope protein. The M9 construct contained the exterior receptor binding domain and membrane-associated domain of VSV G protein, fused to sequences encoding the cytoplasmic domain of MLV envelope protein.

The M2 and M9 constructs were introduced into each of two expression vectors. The first vector, pSVL contained an SV40 late promoter and late protein polyadenylation signal. The second vector, pFR400 contained an SV40 early promoter and an HBV polyadenylation signal. Thus, four expression constructs were produced: pSVLM2, pSVLM9, pFR4M2 and pFR4M9. These expression vectors were introduced into COS cells. Plasmids containing full length VSV G membrane-associated protein (pJM) and full length MLV envelope protein (pSVLENV) were also introduced. Indirect immunofluorescence using rabbit anti VSV G protein was used to analyze VSV G protein immunoreactivity production in these cells. Table 6 gives results from these experiments.

**TABLE 6**

| Transient expression in COS cells | | |
|---|---|---|
| Plasmid | Estimated Percentage Positive Cells | Intensity of fluor. |
| pJM | 44.6%, 17.9% | 1000 |
| pSVLENV | 1.2% | 10 |
| pSVLM2 | 13.3% | 50 |
| pSVLM9 | 10.6% | 60 |
| pFR4M2 | 5.5% | 15 |
| pFR4M9 | 3.6% | 15 |

Full length VSV G proteins from pJM were visible both within the cell and on the cell surface. Hybrid M9 proteins were only visible in periuclear regions. By fluorescent activated cell sorting (FACS) it was determined that only 10% of these cells expressed hybrid M9 protein on their cell surface. The molecular weight of the hybrid proteins were confirmed by immunoprecipitation using Rabbit anti VSV G. The mobilities were consistent with the protein size predicted from the nucleotide sequences.

The results of the experiments of Example 9 indicate that the most efficient packaging resulted from full length VSV G protein, with either of the hybrid proteins with MLV cytoplasmic domains being expressed less efficiently on the cell surface. As discussed above, this finding was unexpected due to the prevailing belief that retroviral cytoplasmic domain was required for efficient vector particle packaging of vector particles having retrovirally derived nucleocapsid. Thus, Example 9 confirms our surprising discovery that enveloped vector particles having nucleocapsids derived from a particular virus can be efficiently packaged with heterologous membrane-associated proteins.

Example 9 also indicates that the VSV G protein cytoplasmic and membrane domains are efficient at directing protein to the cell surface. Thus, it is believed that hybrid protein could be created in a manner similar to the manner of Example 9, wherein the cytoplasmic and membrane domains of VSV G protein are combined to create a cassette for vector particle targeting where exterior receptor domains from a variety of unrelated proteins could be used with the cassette.

As stated above, we believe that the regions of membrane-associated proteins involved with vector particle formation lie chiefly within the membrane-associated domains of the protein. Thus, recombinant chimeric membrane-associated proteins useful in the vector particles of the present invention contain the membrane-associated domain from a membrane-associated protein shown to be efficiently packaged into vector particles. The exterior receptor domains of these chimeric proteins can be selected from any ligand/receptor to determine a host range, as described above. Example 10 is provided as one example of the production of vector particles having a recombinant chimeric membrane-associated protein.

### EXAMPLE 10

### Cassette for Production of Vector Particles having Chimeric Membrane-Associated Protein

The hybrid membrane associated glycoprotein is constructed following guidelines set forth in Example 9. As a model for this example, the VSV G cytoplasmic and membrane domains are genetically recombined with the gene encoding erythropoietin (EPO). This recombinant chimera is inserted in expression vector pSVL from Example 9. In addition, vector pLRNL and vector pSVGP are transfected into producer cells. The resulting supernatant is collected and the vector particles are used to infect B6SutA cells. B6SutA cells contain the EPO receptor and under neomycin selection, only those cells receiving vector particles containing EPO hybrid protein and pLRNL vector particle genome will survive.

The methodology described in Example 10 can be used to create a cassette system in which genes for any desired receptor binding domain can be recombined with the membrane-associated domain from VSV G protein or other membrane-associated domain. Thus, Example 10 provides methods for the rapid production of a variety of vector particles having a host range defined by the receptor binding domain inserted into the cassette.

Vector particles having non-native membrane-associated ligands as described herein, will, advantageously, have a host range determined by the ligand-receptor interaction of the membrane-associated protein. For example, where the membrane associated protein is derived from an enveloped virus capable of infecting human cells, the vectors will be able to introduce foreign genes into human cells with greatly increased efficiency. The neomycin gene used in the previous examples could be substituted with a therapeutic gene that would also be under the control of the RSV promoter. Similarly the therapeutic gene could be introduced in addition to the neomycin gene. As mentioned, there are a number of promoters and marker genes that could be used in this system. Therapeutic genes could take the form of nucleic acid sequences encoding protein or nucleic acid sequences with inherent regulatory value.

Therapeutic genes encoding protein could take the form of polypeptide to supplement a given protein defect, to improve or alter an immune function or one that would render a specific protein or set of proteins inactive. The therapeutic protein could potentially have regulatory activity to stimulate or inhibit a protein or set of proteins. The therapeutic protein could also stimulate or inhibit a given cell function. In other words the therapeutic gene could encode a polypeptide with a range of activities not necessarily limited to this discussion but selectable to those individuals with skill in the art.

The nucleic acid could also code for a product having activity as nucleic acid. For example, this nucleic acid could have antisense activity to prevent translation of a given protein or it could have DNA binding capabilities that could promote or inhibit expression of protein or a set of proteins or promote or inhibit a given cell function. Again, the sequence of choice would be apparent to an individual with skill in the art.

Thus, for targeted gene therapy, a vector particle having altered host range can be produced using the methods of the present invention. The ligand will be selected to provide a host range including the targeted cell type. Thus, in order to provide therapy for a genetic defect in which a polypeptide is not expressed in sufficient quantities or in sufficient quantities of active form, the ligand will be selected so as to target the cell types that normally produce that polypeptide. The gene for the polypeptide should also be introduced under the control of an appropriate viral promoter.

Alternatively, therapy can also be provided by inhibiting the expression of undesired polypeptides, e.g. through the production of anti-sense oligonucleotides which interfere with the translation of mRNA coding for the undesired polypeptides. Example 11 is provided to illustrate the production of a targeted vector particle delivering a therapeutic oligonucleotide for HIV infection.

### EXAMPLE 11

### Delivery Vehicle for Anti-HIV Therapeutic Agent

The vector particle from Example 8 is altered by replacing the Neo gene with a sequence encoding an antisense oligonucleotide for a region complementary to the mRNA of the major HIV protease. The resulting vector particles contain CD4 as a membrane-associated protein and direct the transcription of antisense mRNA that hybridizes with HIV mRNA to restrict HIV replication.

Thus, Example 10 shows a technique wherein a vector particle genome is modified to direct a particular therapy to particular cell types. It is also contemplated that the present invention include the targeting of progenitor cells. For example, there are a number of progenitor cell types found in bone marrow that differentiate into blood cells. Many blood cells have relatively short life spans and therefore progenitor cells continually divide and differentiate to replace the lost cells. Therapies directed to blood cells would be most useful if they target the progenitor cell. These cells are known to have unique cellular determinants that permit histological identification and could be used as cell receptors for the membrane-associated proteins of the vector particles of the present invention. The vectors could be constructed to target these cell types for gene delivery by including an expressible gene which encodes a membrane-associated protein that binds to a unique cellular determinant of such progenitor cell types. Examples of such progenitor cell types which could be targeted using vector particles of the present invention include pluripotent stem cells, erythroblasts, lymphoblasts, myeloblasts and megakaryocytes.

It is further envisioned that the therapy of the present invention be performed either in vivo or in vitro. For in vitro therapy of an organism, cells could be removed and infected with the vector particle in vitro. For vector particles having membrane-associated proteins which determine the appropriate host range, there would be no need to purify the cells to be targeted in vitro because the viral vector would specifically infect the appropriate cells. Thus, bone marrow samples could be removed from a subject and the desired cell type transfected. These transfected cells could then be returned to the organism of origin of the cells or, where rejection of the cells is not envisioned to be a problem, to another organism. The sample could be treated with growth factors in vitro either before or after transfection to increase the number of cells to be either targeted or returned to the subject organism.

In order to further the goals of altering the host range of these viral vectors, we have discovered that the development of stable packaging cell lines constitutively expressing membrane-associated proteins would facilitate even further the generation of retroviral vectors with wider host range and with increased efficiency of infection in human and other cells. Many of these membrane-associated proteins, including the VSV G envelope protein, are toxic to cells. This results in producer line stability problems because expressor cells die quickly, resulting in difficulties in creating more than transient production. Although some cell types (e.g., human 293, its derivative 2-3 which makes gag and pol proteins from MLV, or hamster BHK cells) will grow after transduction with vector particle LGRNL and neo selection, this growth lasts only four weeks and then the cells die. Other cell types die even more rapidly. Thus, the development of stable packaging cell lines is highly desirable.

One method for producing stable packaging cell lines is to develop a packaging construct in which the envelope protein is subject to an inducible promoter in which expression of the envelope protein can be turned on or off through the presence or absence of a particular compound or through a change in conditions such as temperature. One example of such an inducible promoter is the mouse mammary tumor virus (MMTV) promoter. The MMTV promoter is highly inducible by corticosteroid and their analogues in cells which synthesize glucocorticoid receptor proteins in sufficient amounts.

A cell line having the ability to express vector particle genes from an inducible promoter, such as the MMTV promoter, will not produce vector particle without induction by appropriate inducing conditions, e.g. the presence of corticosteroid for the MMTV promoter. Accordingly, a cell line having the ability to produce vector particles can be stably maintained without the production of vector particles. A subpopulation from the cell line could then be induced to produce vector particles by the appropriate conditions when desired. An example of the production of a stable packaging cell line having the ability to express vector particle genes under the control of an inducible promoter is shown in Example 12.

### EXAMPLE 12

### Production of Stable Packaging Construct with Inducible Promoter

In order to identify a cell line which synthesize glucocorticoid receptor proteins in sufficient amounts to provide for induction of the MMTV promoter in the presence of corticosteroid, a construct with the MMTV promoter directing transcription of a marker gene such as the chloramphenicol acetyl transference (CAT) gene is produced. This construct is introduced into various cell lines, with or without dexamethasone treatment.

Those cell lines exhibiting a ratio of CAT activity in the dexamethasone treated cells to that in the untreated cells greater than 25:1 are preferred for use as stable packaging cell lines under the control of the inducible MMTV promoter. A cell line exhibiting such a ratio is the BHK cell line.

To produce a stable packaging cell line for MLV[VSV G], BHK cells are permanently transfected with a packaging construct, pSVGP and subsequently with a construct linking the MMTV promoter to the VSV G gene and to RSV LTR driving the hygromycin resistance gene, as a selectable marker. Hygromycin-resistant cells are selected and checked for Gag and Pol production by Western blots using anti-MLV 30 as probe. These cells are also checked for the presence of the MMTV-VSV G gene by a Southern blot analysis.

Cells that continue to grow (and hence make non-lethal levels of VSV-G) are tested by transient transfection with a vector such as LRNL, treated or not treated with dexamethasone and the ratio of neo resistant colony forming units determined. The cell clones with the best performance are permanently transfected with MLV[VSV G] vector particle producing nucleic acid. Infectious particle production is induced for short periods of time (1-3 days) by dexamethasone treatment.

Thus, Example 12 shows one example of the production of a stable packaging construct under the control of an inducible promoter. Similar techniques can be used to produce a variety of vector particles of the present invention under the control of MMTV promoter or other inducible promoters.

Another example of the production of a stable packaging construct involves the development of cells which can tolerate the production of the membrane-associated protein. One cell line, MDCK, (ATCC No. CCL³⁴) has been reported to be resistant to the toxic effects of VSV and to be capable of supporting long-term production of VSV-G. Other such cell lines are believed to exist. However, not all VSV G protein produced by such cell lines is functional. Accordingly, clones producing functional protein must be selected. An example of the production of stable packaging cell line producing functional membrane-associated protein is shown in Example 13.

### EXAMPLE 13

### Production of Stable Packaging Construct in Tolerated Cell Line

MDCK cells were tested for their tolerance to the stable expression of VSV G membrane associated protein and for their ability to produce vector particles. VSV G tolerance was tested by infecting MDCK cells with MLV[VSV G] vector particles and selecting for G418 resistant clones. The vector particles were produced from the transient transfection of pLGRNL into human 293 cells that stably expressed the retroviral gag and pol proteins. The vector particle-containing supernatant of these transfected cells was used to infect MDCK cells. These vector particles contain pLGRNL as their vector genome and VSV G as their membrane-associated protein.

MDCK cells were infected and the cells were cultured for 24 hours prior to selection in G418. At 24 hours post-infection, the cells were treated with 400 µg/ml of the neomycin analog G418. After about 7-10 days of selection, colonies began to appear that were resistant to G418. When the colonies were clearly visible and separated from non-resistant cells, they were pooled and subcultured in the presence of G418 for further analysis.

The above-described polyclonal cell line was tested for the expression of VSV G from the pLGRNL vector genome. Briefly, the cells were grown to 60-70% confluency and incubated in methionine-free medium without fetal calf serum (Met⁻ DME) for 30 minutes at 37°C. The medium was replaced with 2 mls of Met⁻ DME containing 300 µCi of ³⁵S-methionine and incubated for an additional 4 hours. Cells were washed with ice cold PBS and 1 ml of RIPA lysis buffer (150 mM NaCl, 1% NP-40, 0.5% deoxycholate, 0.19% SDS, 50 mM Tris pH 8.0) containing 10 µl of the protease inhibitor, PMSF, at a concentration of 1 mM was added to each plate. The plates were incubated on ice for 15-30 minutes, followed by a 10 minute centrifugation after transfer of the lysed cell material to a microfuge tube. The supernatant was removed and either stored at -70°C or used directly for immunoprecipitation. These lysates were prepared for immunoprecipitations by first treating with non-immune rabbit serum to pre-clear non-specific binding activity.

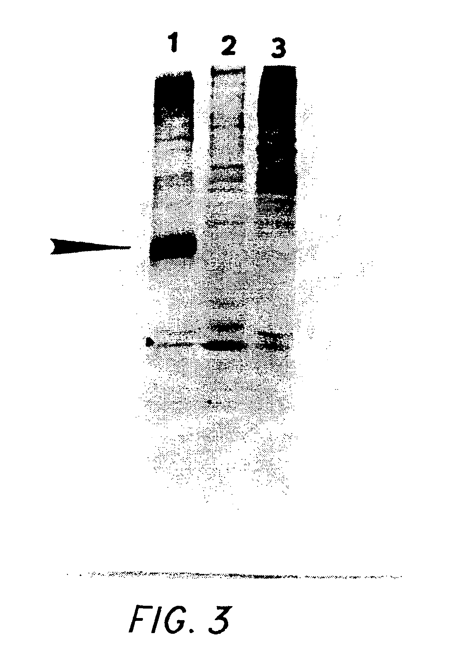

Immunoprecipitations were performed by incubating a volume containing 1x10⁷ counts of pre-cleared lysate with about 5 µl of rabbit anti-VSV G antibody overnight at 4°C. Protein A-Sepharose (100 µl of a 10% solution in RIPA buffer) was then added, followed by incubation for 1-2 hours at 4°C. The conjugates were washed three times in RIPA buffer and the final pellets were resuspended in 30 µl of Laemmli sample buffer. Immunoprecipitates were separated by SDS-PAGE and visualized by fluorography. The results are shown in Figure 3. Lanes 1 and 2 show the polyclonal cell line immunoprecipitated with anti-VSV G and normal rabbit serum, respectively. Lane 3 is mock infected MDCK cells immunoprecipitated with anti-VSV G antibody. The band migrating at Mᵣ of approximately 68 kD corresponds to the VSV G protein and documents that these MDCK cells support the long term expression of VSV G.

The MDCK/VSV G polyclonal cell line was also tested for its ability to generate VSV G-containing vector particles. To do this, the cells were co-transfected with ptkhygro as a selectable marker and pSVGP to supply the gag/pol functions.

The polycation Polybrene (Aldrich, Milwaukee, WI) was used for the co-transfection of MDCK/VSV G cells. Briefly, exponentially growing cells were harvested by trypsinization and replated at 5 x 10⁵ cells per 100 mm dish in 10 mls of medium containing 10% FCS. Cultures were incubated for 18-20 hours at 37°C in an atmosphere of 10% CO₂. Transfection DNA (2 µg of ptkhygro and 18 µg of pSVGp) was mixed with 3 mls of serum-containing medium followed by the addition of 30 µg of Polybrene. This transfection solution was applied to the cells after removal of the medium. Cells were incubated for 10 hours. The DNA medium mixture was aspirated and mixed with 2 ml of a DMSO solution (3 parts DMSO:1 part medium). The cells were incubated for 5 minutes at room temperature. After 3 washes with medium, the cells were incubated for an additional 48 hours in complete medium. At this time, the cells were incubated in selective medium containing hygromycin (400 µg/ml) and G418 allowing for the selection of doubly-resistant cells. Hygromycin-resistant and G418-resistant colonies were pooled as described previously and VSV G-containing vector particle production was titered on BHK cells.

Infection of BHK cells with vector particles derived from the resistant cells yielded about 100-200 G418-resistant colonies per 10 ml of media. This result demonstrated that the MDCK polyclonal cell line could generate functional vector particles containing the VSV-G envelope glycoprotein and the pLGRNL vector genome.

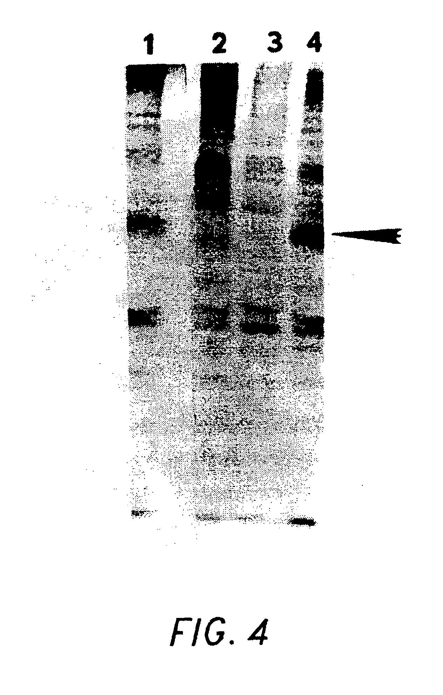

A stable packaging cell line that does not contain a packaging-competent vector particle genome but does stably express VSV-G, gag and pol was then constructed in a two-step transfection procedure. In the first step, pTKhygro and pSVG (VSV G under the control of the SV40 early promoter) were co-transfected using Polybrene into MDCK cells and selected for hydromycin resistance as described previously. The resistant cells were pooled and the cells co-expressing VSV G were isolated by fluorescent activated cell sorting (FACS). Briefly, subconfluent plates were washed once in PBS and then treated for 15 minutes at 37°C with 10 mM EDTA to remove cells from the dish. The cells were transferred to a 15 ml centrifuge tube, pelletted by low speed centrifugation, resuspended in DME plus 2% FCS and re-centrifuged. Media was removed and the pellet was resuspended in 0.5 mls of a 1:200 dilution of normal rabbit serum or a 1:200 dilution of rabbit anti-VSV G antibody. Cells were incubated with the antibody for 30 minutes on ice and then diluted with 5.0 mls of DME plus 2% FCS, followed by low speed centrifugation. The resultant pellets were washed an additional two times with 5 mls of DME plus 2% FCS. The cell pellet was resuspended in a 500 µl PBS solution containing the secondary antibody and incubated on ice for 30 minutes. Following the incubation period, the cells were washed as described above and the final pellet was resuspended in 0.5 ml of DME plus 2% FCS with Pen/Strep and Fungizone and injected into the cell sorter. Selected cells were pooled, grown for 1 week, and then reisolated by FACS sorting. These twice-sorted cells were analyzed for VSV G expression by immunoprecipitation with anti-VSV G antibody as described previously. The results are shown in Figure 4 where lane 1 shows the extracts of the twice-sorted cells immunoprecipitated with the anti-VSV-G antibody and lanes 2, 3 and 4 correspond to lanes 3, 2 and 1 of Figure 3, respectively. These results demonstrate that the pSVG transfected cells stably express substantial levels of VSV G.

The second step in producing the stable packaging line was to co-transfect pSVGP and a plasmid that expresses dihydrofolate reductase (DHFR) to achieve the stable expression of the gag and pol genes. DHFR was used for the selection of stable transfectants when grown in the presence of methotrexate (MTX). The plasmids were co-transfected as described previously using Polybrene and replated at 48 hours post transfection in the presence of 5 µM MTX. MTX-resistant colonies are pooled and tested for the generation of vector particles by transient transfection of these cells with a packaging competent vector genome. Supernatants are collected at 48 hours and the production of infectious vector is titered on target BHK cells as described previously.

Thus, Example 13 shows the production of a permanent VSV G packaging line by selective screening for functional VSV G protein production in cell lines resistant to the toxic effect of VSV G protein. As will be readily apparent to those of ordinary skill in the art, similar methods can be used to create cell lines which produce functional membrane-associated protein from a variety of sources.

## Claims

1. A mammalian packaging cell containing nucleotide sequences encoding gag, pol, and a heterologous chimeric membrane-associated protein which determines a host range, the chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G and wherein the exterior receptor binding domain is selected from any ligand/receptor to determine a host range which is different from that of the virus from which the nucleotide sequence encoding gag and pol derives.

2. The cell of claim 1, wherein said chimeric protein is derived from two origins and no more than one of said two origins is retroviral.

3. The cell of claim 1, wherein the exterior receptor binding domain is that of erythropoietin.

4. A recombinant vector particle comprising:
gag and pol retroviral proteins;
a nucleic acid sequence associated with the vector particle; and
an envelope comprising a heterologous chimeric membrane-associated protein which determines a host range, the chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G, and wherein the exterior receptor binding domain is selected from any ligand/receptor to determine a host range which is different from that of the virus from which the gag and pol retroviral protein derive.

5. The recombinant vector particle of claim 4, wherein said chimeric protein is derived from two origins and no more then one of said two origins is retroviral.

6. The recombinant vector particle of claim 4, wherein the exterior receptor binding domain is that of erythropoietin.

7. A method for producing a recombinant enveloped vector particle from a mammalian packaging cell, the recombinant virion comprising a heterologous chimeric membrane-associated protein, the method comprising the steps of:
introducing a retroviral vector into a mammalian packaging cell, the cell stably expressing gag and pol, and expressing a heterologous chimeric membrane-associated protein which determines a host range, said chimeric membrane-associated protein comprising an exterior receptor binding domain, a membrane-associated domain, and a cytoplasmic domain, wherein the membrane-associated domain is the membrane-associated domain of VSV G and the cytoplasmic domain is the cytoplasmic domain of VSV G, and wherein the exterior receptor binding domain is selected from any ligand/receptor to determine a host range which is different from that of the virus from which gag and pol derive;
culturing the mammalian packaging cell; and
isolating a recombinant enveloped vector particle produced by the cell.

8. A stable mammalian packaging cell stably expressing a first nucleotide sequence encoding retroviral gag and pol and inducibly expressing a second heterologous nucleotide sequence encoding VSV G which determines a host range being different from that of the retrovirus from which said first nucleotide sequence derives, wherein said second nucleotide sequence is operably linked to an inducible promoter.

9. The packaging cell of claim 8, wherein the inducible promoter is an MMTV promoter.

10. The packaging cell of claim 8, wherein the cell is a BHK cell.

11. A method of producing an enveloped vector particle from a mammalian packaging cell line, the method comprising the steps of:
introducing a retroviral vector into a stable mammalian packaging cell containing a first nucleotide sequence encoding gag and pol, and a second nucleotide sequence encoding VSV G, which determines a host range being different from that of the retrovirus from which said first nucleotide sequence derives, said second nucleotide sequence being operably linked to an inducible promoter;
culturing the cell under conditions for induction of expression of VSV G from said inducible promoter; and
isolating vector particles produced by the cell.

12. The method of claim 11, wherein expression from the inducible promoter is temperature dependent.

13. The method of claim 11, wherein expression from the inducible promoter is dependent upon the presence of at least one compound.

14. The method of claim 13, wherein the inducible promoter is MMTV and the compound is corticosteroid.

## Patentansprüche

1. Säuger-Verpackungszelle, die Nukleotidsequenzen, welche für gag, pol codieren, und ein heterologes, chimäres, membranassoziiertes Protein enthält, welches ein Wirtsspektrum festlegt, wobei das chimäre, membranassoziierte Protein eine äußere Rezeptorbindedomäne, eine membranassoziierte Domäne und eine cytoplasmatische Domäne umfasst, wobei die membranassoziierte Domäne die membranassoziierte Domäne von VSV G ist und die cytoplasmatische Domäne die cytoplasmatische Domäne von VSV G ist, und wobei die äußere Rezeptorbindedomäne ausgewählt ist aus irgendeinem Liganden/Rezeptor, um ein Wirtsspektrum festzulegen, das verschieden ist von dem des Virus, aus dem die Nukleotidsequenzen stammen, die für gag und pol codieren.

2. Die Zelle nach Anspruch 1, wobei das besagte chimäre Protein aus zwei Quellen stammt und nicht mehr als eine der besagten zwei Quellen retroviral ist.

3. Die Zelle nach Anspruch 1, wobei die äußere Rezeptorbindedomäne die von Erythropoietin ist.

4. Rekombinantes Vektorpartikel, umfassend:
retrovirale gag- und pol-Proteine;
eine Nukleinsäuresequenz, die mit dem Vektorpartikel assoziiert ist; und
eine Hülle, die ein heterologes, chimäres, membranassoziiertes Protein umfasst, welches ein Wirtsspektrum festlegt, wobei das chimäre, membranassoziierte Protein eine äußere Rezeptorbindedomäne, eine membranassoziierte Domäne und eine cytoplasmatische Domäne umfasst, wobei die membranassoziierte Domäne die membranassoziierte Domäne von VSV G ist und die cytoplasmatische Domäne die cytoplasmatische Domäne von VSV G ist, und wobei die äußere Rezeptorbindedomäne ausgewählt ist aus irgendeinem Liganden/Rezeptor, um ein Wirtsspektrum festzulegen, das verschieden ist von dem des Virus, aus dem die retroviralen gag- und pol-Proteine stammen.

5. Rekombinantes Vektorpartikel nach Anspruch 4, wobei das besagte chimäre Protein aus zwei Quellen stammt und nicht mehr als eine der besagten zwei Quellen retroviral ist.

6. Rekombinantes Vektorpartikel nach Anspruch 4, wobei die äußere Rezeptorbindedomäne die von Erythropoietin ist.

7. Verfahren zur Herstellung eines rekombinanten, umhüllten Vektorpartikels aus einer Säuger-Verpackungszelle, wobei das rekombinante Virion ein heterologes, chimäres, membranassoziiertes Protein umfasst, wobei das Verfahren die Schritte umfasst:
Einbringen eines retroviralen Vektors in eine Säuger-Verpackungszelle, wobei die Zelle gag und pol stabil exprimiert, und Exprimieren eines heterologen, chimären, membranassoziierten Proteins, das eine Wirtsspektrum festlegt, wobei das besagte chimäre, membranassoziierte Protein eine äußere Rezeptorbindedomäne, eine membranassoziierte Domäne und eine cytoplasmatische Domäne umfasst, wobei die membranassoziierte Domäne die membranassoziierte Domäne von VSV G ist und die cytoplasmatische Domäne die cytoplasmatische Domäne von VSV G ist, und wobei die äußere Rezeptorbindedomäne ausgewählt ist aus irgendeinem Liganden/Rezeptor, um ein Wirtsspektrum festzulegen, das verschieden ist von dem des Virus, aus dem gag und pol stammen;
Inkulturbringen der Säuger-Verpackungszelle; und
Isolieren eines rekombinanten, umhüllten Vektorpartikels, das von der Zelle hergestellt wird.

8. Stabile Säuger-Verpackungszelle, die eine erste Nukleotidsequenz stabil exprimiert, welche für retrovirales gag und pol codiert, und die eine zweite heterologe Nukleotidsequenz induzierbar exprimiert, die für VSV G codiert, welches ein Wirtsspektrum festlegt, das verschieden ist von dem des Retrovirus, aus dem die besagte erste Nukleotidsequenz stammt, wobei die besagte zweite Nukleotidsequenz mit einem induzierbaren Promotor funktional verbunden ist.

9. Verpackungszelle nach Anspruch 8, wobei der induzierbare Promotor ein MMTV-Promotor ist.

10. Verpackungszelle nach Anspruch 8, wobei die Zelle eine BHK-Zelle ist.

11. Verfahren der Herstellung eines umhüllten Vektorpartikels aus einer Säuger-Verpackungszelllinie, wobei das Verfahren die Schritte umfasst:
Einbringen eines retroviralen Vektors in eine stabile Säuger-Verpackungszelle, der eine erste Nukleotidsequenz, die für gag und pol codiert, und eine zweite Nukleotidsequenz enthält, die für VSV G codiert, welches ein Wirtsspektrum festlegt, das verschieden ist von dem des Retrovirus, aus dem die besagte erste Nukleotidsequenz stammt, wobei die besagte zweite Nukleotidsequenz mit einem induzierbaren Promotor funktional verbunden ist;
Inkulturbringen der Zelle unter Bedingungen zur Induktion der Expression von VSV G über den besagten induzierbaren Promotor; und
Isolieren der Vektorpartikel, die von der Zelle hergestellt werden.

12. Verfahren nach Anspruch 11, wobei die Expression über den induzierbaren Promotor temperaturabhängig ist.

13. Verfahren nach Anspruch 11, wobei die Expression über den induzierbaren Promotor von dem Vorliegen von mindestens einer Verbindung abhängig ist.

14. Verfahren nach Anspruch 13, wobei der induzierbare Promotor MMTV ist und die Verbindung Corticosteroid ist.

## Revendications

1. Cellule d'empaquetage de mammifère, contenant des séquences nucléotidiques codant gag, pol et une protéine chimère hétérologue associée à la membrane, qui détermine une gamme d'hôtes, laquelle protéine chimère associée à la membrane comprend un domaine extérieur de liaison au récepteur, un domaine associé à la membrane et un domaine cytoplasmique, le domaine associé à la membrane étant le domaine associé à la membrane de VSV G et le domaine cytoplasmique étant le domaine cytoplasmique de VSV G, et le domaine extérieur de liaison au récepteur étant choisi parmi n'importe quels ligands/récepteurs de manière à déterminer une gamme d'hôtes qui soit différente de celle du virus dont est issue la séquence nucléotidique codant gag et pol.

2. Cellule conforme à la revendication 1, dans laquelle la protéine chimère dérive de deux origines, dont au plus l'une est rétrovirale.

3. Cellule conforme à la revendication 1, dans laquelle le domaine extérieur de liaison au récepteur est celui de l'érythropoïétine.

4. Particule vecteur recombinée comprenant :
- les protéines rétrovirales gag et pol ;
- une séquence d'acide nucléique associée à la particule vecteur ;
- et une enveloppe comprenant une protéine chimère hétérologue associée à la membrane, qui détermine une gamme d'hôtes, laquelle protéine chimère associée à la membrane comprend un domaine extérieur de liaison au récepteur, un domaine associé à la membrane et un domaine cytoplasmique, le domaine associé à la membrane étant le domaine associé à la membrane de VSV G et le domaine cytoplasmique étant le domaine cytoplasmique de VSV G, et le domaine extérieur de liaison au récepteur étant choisi parmi n'importe quels ligands/récepteurs de manière à déterminer une gamme d'hôtes qui soit différente de celle du virus d'où sont issues les protéines rérrovirales gag et pol.

5. Particule vecteur recombinée conforme à la revendication 4, dans laquelle la protéine chimère dérive de deux origines, dont au plus l'une est rétrovirale.

6. Particule vecteur recombinée conforme à la revendication 4, dans laquelle le domaine extérieur de liaison au récepteur est celui de l'érythropoïétine.

7. Procédé de préparation d'une particule vecteur à enveloppe recombinée, à partir d'une cellule d'empaquetage de mammifère, le virion recombiné comprenant une protéine chimère hétérologue associée à la membrane, lequel procédé comporte les étapes suivantes :
- introduire un vecteur rétroviral dans une cellule d'empaquetage de mammifère, laquelle cellule exprime de manière stable gag et pol, et exprime une protéine chimère hétérologue associée à la membrane, qui détermine une gamme d'hôtes, laquelle protéine chimère associée à la membrane comprend un domaine extérieur de liaison au récepteur, un domaine associé à la membrane et un domaine cytoplasmique, le domaine associé à la membrane étant le domaine associé à la membrane de VSV G et le domaine cytoplasmique étant le domaine cytoplasmique de VSV G, et le domaine extérieur de liaison au récepteur étant choisi parmi n'importe quels ligands/récepteurs de manière à déterminer une gamme d'hôtes qui soit différente de celle du virus d'où sont issues gag et pol;
- cultiver la cellule d'empaquetage de mammifère ;
- et isoler une particule vecteur à enveloppe recombinée produite par la cellule.

8. Cellule d'empaquetage stable de mammifère, exprimant de façon stable une première séquence nucléotidique codant les gag et pol rétrovirales et exprimant de manière inductible une deuxième séquence nucléotidique hétérologue codant VSV G, qui détermine une gamme d'hôtes qui est différente de celle du rétrovirus d'où est issue ladite première séquence nucléotidique, ladite deuxième séquence nucléotidique étant opérationnellement liée à un promoteur inductible.

9. Cellule d'empaquetage conforme à la revendication 8, dans laquelle le promoteur inductible est un promoteur de MMTV.

10. Cellule d'empaquetage conforme à la revendication 8, laquelle cellule est une cellule BHK.

11. Procédé de production d'une particule vecteur à enveloppe à partir d'une lignée cellulaire d'empaquetage de mammifère, lequel procédé comprend les étapes suivantes :
- introduire un vecteur rétroviral dans une cellule d'empaquetage stable de mammifère, contenant une première séquence nucléotidique codant gag et pol et une deuxième séquence nucléotidique codant VSV G, qui détermine une gamme d'hôtes qui est différente de celle du rétrovirus d'où est issue ladite première séquence nucléotidique, ladite deuxième séquence nucléotidique étant opérationnellement liée à un promoteur inductible ;
- cultiver la cellule dans des conditions permettant l'induction de l'expression de VSV G à partir dudit promoteur inductible ;
- et isoler les particules vecteurs produites par la cellule.

12. Procédé conforme à la revendication 11, dans lequel l'expression à partir du promoteur inductible dépend de la température.

13. Procédé conforme à la revendication 11, dans lequel l'expression à partir du promoteur inductible dépend de la présence d'au moins un composé.

14. Procédé conforme à la revendication 13, dans lequel le promoteur inductible est MMTV et le composé est un corticostéroïde.
